# EUROPÄISCHE PATENTSCHRIFT

(11) **EP 1 021 192 B1**
(45) Veröffentlichungstag und Bekanntmachung des Hinweises auf die Patenterteilung: **08.01.2003**
(21) Anmeldenummer: 98954349.1
(22) Anmeldetag: 05.10.1998
(51) Int. Cl.: A61K 31/57, A61K 47/32

(54) **DEXAMETHASON-GEL**
DEXAMETHASONE GEL
GEL DE DEXAMETHASONE

(30) Priorität: 06.10.1997 DE 19744113
(43) Veröffentlichungstag der Anmeldung: 26.07.2000
(73) Patentinhaber: Dr. GERHARD MANN chem.-pharm. Fabrik GmbH, D-13581 Berlin (DE)
(72) Erfinder: BELLMANN, Günther, D-13593 Berlin (DE); CLAUS-HERZ, Gudrun, D-14167 Berlin (DE); KESSLER, Christoph, D-10625 Berlin (DE)
(74) Vertreter: Maiwald, Walter, Dr. Dipl.-Chem.
(86) Internationale Anmeldenummer: EP9806339
(87) Internationale Veröffentlichungsnummer: WO99017780

(56) Entgegenhaltungen:
- EP-A- 0 028 110
- EP-A- 0 275 054
- EP-A- 0 801 948
- WO-A-93/17664
- WO-A-95/05803
- WO-A-97/00669
- DE-C- 4 404 990
- GB-A- 2 007 091
- US-A- 4 271 143
- US-A- 5 252 318
- US-A- 5 397 567

## Beschreibung

Die Erfindung betrifft ein ophthalmologisches Präparat mit einem Gehalt von Dexamethason Dihydrogenphosphat-Dinatrium als Wirkstoff, sowie gegebenenfalls Gehalten an üblichen Zusatzstoffen und Wasser.

Dexamethason-Präparate sind in Form von Augentropfen und Augensalben bekannt. Augentropfen haben üblicherweise Konzentrationen zwischen 0,05 und 0,1 %, während Augensalben üblicherweise etwa 0,05 % Dexamethason-Natriumphosphat enthalten.

Neben Augentropfen und Augensalben sind auch gelartige ophthalmische Formulierungen mit unterschiedlichen Wirkstoffen im Stand der Technik bekannt.

Beispielsweise wird in der GB 2 007 091 eine ophthalmische Zusammensetzung in Form eines Gels beschrieben, welche eine wäßrige Lösung eines Carboxyvinylpolymers sowie einen Wirkstoff umfaßt und das resultierende gelartige Präparat einen pH-Wert im physiologisch tolerierbaren Bereich aufweist.

Die US 4,271,143 betrifft eine ophthalmische Gelzusammensetzung, welche neben einem ophthalmischen Wirkstoff ein hochmolekulares Polymer zur Ausbildung eines hochviskosen Gels umfaßt. Auch in der US 4,271,143 wird angegeben, daß das entsprechende Gelpräparat einen physiologisch tolerierbaren Wert im Bereich von pH 4,5 bis pH 8,5 aufweisen sollte. Spezifische bzw. bevorzugte pH-Wertbereiche oder pH-Werte werden nicht genannt.

Die WO97/0066 offenbart eine stabilisierte Zusammensetzung, die sich aus mindestens einem Polymer, einem stark gelatisierenden Agens und einem ophthalmischen Wirkstoff zusammensetzt. Gemäß der Lehre der WO97/00669 verhindert der Zusatz des stark gelatisierenden Agens eine Zersetzung der ophthalmischen Zusammensetzungen und ermöglicht somit längere Lagerzeiten des Präparats.

Die WO93/17664 offenbart ophthalmische Zusammensetzungen, die aus einem Zellulosepolymer und einem Carboxyvinylpolymer sowie einem pharmazeutisch aktiven Agens bestehen. Angaben zur pH-Werteinstellung des Präparats werden in der WO93/17664 nicht gemacht.

Die EP 0 028 110 betrifft ophthalmische Präparate, die auf einer Öl-in-Wasser-Emulsion basieren.

EP 0 275 054 offenbart Zusammensetzungen, die eine wäßrige Carboxyvinylpolymerlösung, eine bestimmte Menge an Aminosäure und einen pharmazeutischen Wirkstoff umfassen. Auch in diesem Dokument des Standes der Technik wird lediglich eine undifferenzierte Bereichsangabe zum pH-Wert des ophthalmischen Präparates gemacht.

Die bekannten Augentropfenformulierungen mit Dexamethason-Estem als Wirkstoff werden auf leicht alkalische pH-Werte eingestellt. So haben gegenwärtig im Handel erhältliche Tropflösungen typischerweise einen pH-Wert von etwa 7,3. Diese leicht alkalischen pH-Werte werden gewählt, weil Dexamethason-Ester, ebenso wie Prednisolon-Ester, im leicht alkalischen Bereich die beste Stabilität aufweisen.

Dabei wählt man typischerweise den pH-Wert möglichst nahe am Neutralpunkt, weil höhere Alkalinitäten das Auftreten von Augenreizungen und anderen Verträglichkeitsproblemen befürchten lassen.

Es ist bekannt, daß die antiphlogistische Wirksamkeit von Augentropfen besser ist als die von Augensalben. Die Bioverfügbarkeit aus Augensalben ist, im Experiment, deutlich schlechter gewesen als die aus Augentropfen-Lösung, auch bei längerer Kontaktzeit (Cox et al., Arch. Ophthalmol. 88, 549 (1972); Kupfennan et al., Arch. Ophthalmol. 91, 373 (1974)).

Obwohl Augentropfen eine gegenüber Salben bessere Bioverfügbarkeit zeigen, ist es oft schwierig, beim Einsatz von Tropflösungen die gewünschte Verweilzeit zu realisieren. Tropflösungen werden beispielsweise relativ schnell durch Tränenflüssigkeit ausgeschwemmt, so daß die Konzentration des Wirkstoffs relativ schnell abnimmt.

Für viele Anwendungen wäre es vorteilhaft, eine länger andauernde Konzentration des Wirkstoffs am Auge nach einmaliger Applikation zu realisieren. Dabei kann, wegen der bekannten Bioverfügbarkeitsprobleme, auf Salben nur unter Inkaufnahme erheblicher Nachteile zurückgegriffen werden.

Aufgabe der Erfindung ist es, ein ophthalmologisches Präparat bereitzustellen, das diese Schwierigkeiten überwindet.

Zur Lösung dieser Aufgabe dienen die im beigefügten Hauptanspruch definierten Merkmale.

Vorteilhafte Weiterentwicklungen und Ausgestaltungen sind in den Unteransprüchen definiert.

Der Versuch, ausgehend von den bekannten Tropflösungen eine Gelpräparation im erfindungsgemäßen Sinne dadurch zu formulieren, daß man einen geeigneten Gelbildner hinzufügt, führt zu überraschenden Schwierigkeiten.

Wenn man von den bekannten Tropflösungen im pH-Bereich 7 bis etwa 7,3 ausgeht, führt der Zusatz von Carbomer überraschenderweise zu erheblicher Zersetzung des Wirkstoffes bereits nach kurzer Zeit, so daß die erforderliche Stabilität nicht erreicht wird.

Anscheinend kommt es zu einer verstärkten Umwandlung von Dexa-methason-Dihydrogenphosphat-Dinatrium ("Dexamethason-Natriumphosphat") in die freie Base.

Überraschenderweise kann diese Zersetzung dadurch verhindert werden, daß ein höherer pH-Wert gewählt wird. Gelbildner wie Carbopol scheinen die Haltbarkeit wäßriger Dexamethason-Phosphat-Lösungen im sauren bis neutralen Bereich wie auch im leicht alkalischen Bereich negativ zu beeinflussen, überraschenderweise aber im höher alkalischen Bereich deutlich zu verbessern.

Setzt man daher Carbomere, insbesondere vom Typ Carbopol 980 NF oder ähnliche Carbopol-Produkte, als Gelbildner in erfindungsgemäßen Gelen ein, ergibt sich im pH-Bereich oberhalb 7,3 die gewünschte Stabilität.

Bei Gehalten von 0,05 bis etwa 1 Gew.-%, vorzugsweise zwischen 0,1 und 0,6 Gew.-% und insbesondere bevorzugt bei etwa 0,3 Gew.-% Carbomer, insbesondere vom Typ Carbopol 980 NF, und Wirkstoffkonzentrationen im Bereich von etwa 0,1 Gew.-%, ergeben sich lagerfähige, stabile Gel-Präparate bei pH-Werten oberhalb von 7,3, vorzugsweise oberhalb 7,6 und besonders bevorzugt bei etwa 7,8 und darüber. Die Obergrenze ergibt sich durch die ophthalmologische Verträglichkeit des Präparates, da sehr hohe Alkalinitäten zu Reizungen führen können. In der Praxis wird man daher pH-Werte oberhalb 8 nicht oder nur in besonderen Fällen verwenden.

Das erfindungsgemäße Präparat wird üblicherweise mit pharmakologisch akzeptablen Alkalien, beispielsweise Natriumhydroxid, auf den gewünschten pH-Wert eingestellt.

Es enthält neben dem Wirkstoff, dem Gelbildner und der Base zur Alkalinitätseinstellung noch einen Konservierungsstoff. wie insbesondere Benzododeciniumchlorid (BAC C12), aus der Klasse der Benzalkoniumchloride. Weiterhin enthält das Präparat bevorzugt ein Isotonisierungsmittel, wie Sorbitol, und einen Komplex-Bildner. wie Natriumedetat. Als Lösungsmittel für das Präparat dient Wasser.

Eine typische erfindungsgemäße Rezeptur enthält, für eine Ansatzgröße von 100 kg, etwa die folgenden Bestandteile:

| Bezeichnung | Menge |
|---|---|
| Dexamethason-Dihydrogenphosphat-Dinatrium | 0,0985 kg |
| Benzododeciniumchlorid (BAC C12) | 0,0100 kg |
| Carbopol 980 NF | 0,3000 kg |
| Sorbitol | 4,9000 kg |
| Natriumhydroxid, fest | 0,1460-0,1540 kg |
| Natriumedetat | 0,0100 kg |
| Wasser für Injektionszwecke | 94,5275-94,5355 kg |
| | 100,000 kg |

Überraschenderweise ist ein solches Präparat so haltbar wie eine vergleichbare Tropflösung, also zwei bzw. drei Jahre. Das Präparat ist bei guter Akzeptanz anwendbar, führt nicht zu Augenreizimgen (wozu auch die Wahl des Konservierungsmittels beiträgt) und ergibt die gewünschte längere Verweilzeit bei nicht beeinträchtigter Bioverfügbarkeit des Wirkstoffes, im Vergleich mit Tropflösungen.

## Patentansprüche

1. Ophthalmologisches Präparat mit einem Gehalt an Dexamethason-Dihydrogenphosphat-Dinatrium als Wirkstoff, sowie gegebenenfalls Gehalten an üblichen Zusatzstoffen und Wasser,
**dadurch gekennzeichnet, daß** das Präparat wenigstens eine gelbildende pharmakologisch verträgliche Substanz in einer ausreichenden Menge zur Viskositätseinstellung des Präparates als Gel enthält, wobei das Präparat einen pH-Wert oberhalb 7,3 aufweist.

2. Präparat nach Anspruch 1,
**dadurch gekennzeichnet, daß** das Präparat einen pH-Wert zwischen 7,6 und 8,2 aufweist.

3. Präparat nach Anspruch 2,
**dadurch gekennzeichnet, daß** das Präparat einen pH-Wert zwischen 7,8 und 8,0 aufweist.

4. Präparat nach einem der Ansprüche 1 bis 3,
**dadurch gekennzeichnet, daß** das Präparat wenigstens ein Carbomer, insbesondere vom Typ Carbopol 980 NF, als Gelbildner enthält.

5. Präparat nach Anspruch 4,
**dadurch gekennzeichnet, daß** das Präparat 0,05 bis 1 Gew.-%, vorzugsweise zwischen 0,1 und 0,6 Gew.-% und besonders bevorzugt etwa 0,3 Gew.-% Carbomer enthält.

6. Präparat nach einem der Ansprüche 1 bis 5,
**dadurch gekennzeichnet, daß** das Präparat Dexamethason-Dihydrogenphosphat-Dinatrium in einer Konzentration im Bereich von 0,1 Gew.-% enthält.

7. Präparat nach einem der Ansprüche 1 bis 6,
**dadurch gekennzeichnet, daß** das Präparat als Konservierungsmittel Benzododeciniumchlorid (BAC C12) enthält.

## Claims

1. An ophthalmological preparation having a content of dexamethason dihydrogenphosphate disodium as the active agent, and optionally comprising amounts of commonly used additives and water,
**characterized in that** the preparation includes at least one gel-forming pharmacologically acceptable substance in an amount sufficient for adjusting the viscosity of the preparation to become a gel, wherein the preparation has a pH-value above 7,3.

2. The preparation according to claim 1,
**characterized in that** the preparation has a pH value between 7,6 and 8,2.

3. The preparation according to claim 2,
**characterized in that** the preparation has a pH-value between 7,8 and 8,0.

4. The preparation according to anyone of claims 1 to 3,
**characterized in that** the preparation includes at least one carbomer, in particular of the type carbopol 980 NF, as the gel-forming agent.

5. The preparation according to claim 4,
**characterized in that** the preparation includes 0,05 to 1 weight %, preferably between 0,1 and 0,6 weight % and especially preferred about 0,3 weight % of carbomer.

6. The preparation according to anyone of claims 1 to 5,
**characterized in that** the preparation includes dexamethason dihydrogenphosphate disodium in a concentration in the range of 0,1 % by weight.

7. The preparation according to anyone of claims 1 to 6,
**characterized in that** the preparation includes benzododecinium chloride (BAC-C12) as a preservative.

## Revendications

1. Préparation ophtalmique contenant comme principe actif du dexaméthasone phosphate sodique et, éventuellement des adjuvants usuels et de l'eau,
**caractérisée en ce que** la préparation contient au moins un agent gélifiant pharmacologiquement compatible en quantité suffisante pour conférer à la préparation la viscosité d'un gel, la préparation présentant un pH supérieur à 7,3.

2. Préparation selon la revendication 1,
**caractérisée en ce qu'**elle présente un pH compris entre 7,6 et 8,2.

3. Préparation selon la revendication 2,
**caractérisée en ce qu'**elle présente un pH compris entre 7,8 et 8,0.

4. Préparation selon l'une des revendications 1 à 3, **caractérisée en ce qu'**elle contient en tant qu'agent gélifiant au moins un carbomère, en particulier de type Carbopol 980 NF.

5. Préparation selon la revendication 4,
**caractérisée en ce qu'**elle contient 0,05 à 1 % en poids, de préférence 0,1 à 0,6 % en poids et de manière encore plus préférentielle environ 0,3 % en poids de carbomère.

6. Préparation selon l'une des revendications 1 à 5, **caractérisée en ce qu'**elle contient environ 0,1 % en poids de dexaméthasone phosphate sodique.

7. Préparation selon l'une des revendications 1 à 6, **caractérisée en ce qu'**elle contient comme conservateur du chlorure de benzododécinium (BAC C12).
